# EUROPEAN PATENT APPLICATION

(11) **EP 4 706 537 A1**
(43) Date of publication of application: **11.03.2026**
(21) Application number: 24928949.7
(22) Date of filing: 15.03.2024
(51) Int. Cl.: A61B 5/25

(54) **MEASUREMENT ELECTRODE AND WEARABLE DEVICE**

(71) Applicant: Shenzhen Shokz Co., Ltd., Shenzhen, Guangdong 518108 (CN)
(72) Inventor: SU, Qi, Shenzhen Guangdong 518108 (CN); ZHOU, Xin, Shenzhen Guangdong 518108 (CN); SU, Lei, Shenzhen Guangdong 518108 (CN); LI, Meiqi, Shenzhen Guangdong 518108 (CN); LIAO, Fengyun, Shenzhen Guangdong 518108 (CN); QI, Xin, Shenzhen Guangdong 518108 (CN)
(74) Representative: Wang, Bo
(86) International application number: PCT/CN2024/082039
(87) International publication number: WO 2025/189478

(57) **Abstract**

The present disclosure discloses a measurement electrode and a wearable device. The measurement electrode includes a bottom layer, an electrode layer, and a pad. The bottom layer and the electrode layer are stacked. The pad is disposed between the bottom layer and the electrode layer, both an area of the electrode layer and an area of the bottom layer are greater than an area of the pad. The bottom layer and the electrode layer cover the pad. Through the above manner, the measurement electrode of the present disclosure can better conform to the skin and reduce motion artifacts.

## Description

### TECHNICAL FIELD

The present disclosure relates to the field of wearable device technology, and in particular, to a measurement electrode and a wearable device.

### BACKGROUND

With the improvement of people's living standards and the increasing attention paid to sports, the monitoring of physiological signals has become an indispensable and important health monitoring way. By means of wearable physiological signal monitoring devices, people may monitor their physical conditions anytime and anywhere. A detection of the physiological signals usually requires sensors (e.g., a measurement electrode) to realize. In related technologies, a poor conformity of the measurement electrode to the skin may lead to problems such as motion artifacts.

### SUMMARY

Embodiments of the present disclosure provide a measurement electrode and a wearable device, which enable the measurement electrode to better conform to the skin, thereby addressing the technical problem of motion artifacts generated during measurement.

In a first aspect, embodiments of the present disclosure provide a measurement electrode. The measurement electrode includes a bottom layer, an electrode layer, and a pad. The pad is disposed between the bottom layer and the electrode layer, both an area of the electrode layer and an area of the bottom layer are greater than an area of the pad. The bottom layer and the electrode layer sealedly cover the pad.

In some embodiments, the pad comprises a first main surface and a second main surface disposed opposite to each other, and a side peripheral surface connecting the first main surface and the second main surface.

The electrode layer includes a first covering region, a second covering region, and a bonding region.

The first covering region covers the first main surface, the second covering region covers the side peripheral surface, the bottom layer covers the second main surface, and the bonding region is bound with the bottom layer.

In some embodiments, in a direction away from the bottom layer, the electrode layer includes a waterproof film, a conductive fabric, and a conductive coating sequentially arranged in a stacked arrangement.

In some embodiments, a hardness of the conductive coating is between 0degrees and 50 degrees according to the Shore A standard; a thickness of the conductive coating is 0.01 mm to 0.5mm; and a thickness of the conductive fabric is 0.05 mm to 1 mm.

In some embodiments, the conductive coating is formed by at least one of conductive resin, conductive silicone, conductive thermoplastic polyurethane, or conductive silver paste.

In some embodiments, the conductive resin includes epoxy resin, conductive filler, silane coupling agent, curing agent, and accelerator.

The conductive silicone includes silicone, conductive filler, silane coupling agent, curing agent, and accelerator.

The conductive thermoplastic polyurethane includes thermoplastic polyurethane and conductive filler.

The conductive silver paste includes epoxy resin, silver nanoparticles, curing agent, and accelerator.

In some embodiments, the conductive filler is formed by at least one of conductive carbon black, conductive graphene, or conductive carbon nanotube.

In some embodiments, the conductive coating is coated on a portion of the conductive fabric that covers the pad.

In some embodiments, the conductive fabric includes at least one of conductive silver fabric, nickel-copper conductive fabric, copper conductive fabric, or stainless steel conductive fabric.

In some embodiments, a thickness of the waterproof film is 10 µm to 200 µm.

In some embodiments, the waterproof film is adhered to the conductive fabric through a hot-pressing process, and no adhesive is provided between the waterproof film and the conductive fabric.

In some embodiments, the measurement electrode further includes a terminal, the terminal is disposed on a portion of the electrode layer that does not cover the pad.

In some embodiments, the terminal is disposed on a portion of the conductive fabric that does not cover the pad. The portion of the conductive fabric that does not cover the pad is not provided with the conductive coating. The terminal includes a conductive wire and a composite layer, the conductive wire is disposed between the composite layer and the conductive fabric.

In some embodiments, the conductive wire includes an adhering portion and a lead-out portion. The adhering portion adheres to the electrode layer and is arranged in a serpentine arrangement, and the lead-out portion is connected to the adhering portion and extends outside the electrode layer.

In some embodiments, the composite layer includes a conductive wire layer and a thermoplastic polyurethane layer, the conductive wire layer facing the conductive wire.

In some embodiments, the conductive wire layer includes at least one of a conductive yarn, a conductive metal wire, or a conductive carbon fiber. A diameter of the conductive yarn is 10 to 500 Denier. A diameter of the conductive metal wire is 10 µm to 100 µm, or a diameter of the conductive carbon fiber is 10 µmto 100 µm.

In some embodiments, the pad is formed by at least one of room temperature vulcanized silicone rubber, space cotton, or foam..

In some embodiments, a thickness of the pad is 0.1 mm to 10 mm. A hardness of the pad is between 0 degrees according to the Shore 00 standard and 90 degrees according to the Shore A standard.

In a second aspect, one or more embodiments of the present disclosure provide a wearable device. The wearable device includes the measurement electrode described above.

Advantageous effects of the present disclosure are as follows: different from the situation in the prior field, by disposing a pad with a thickness greater than that of the electrode layer between the electrode layer and the bottom layer, the measurement electrode can have sufficient deformation margin in the thickness direction during contact with the skin, thereby enabling the measurement electrode to better conform to the skin. The conformity of the measurement electrode with the skin can be improved by the above-mentioned manner, thereby addressing the technical problem of motion artifacts generated during the measurement process.

### BRIEF DESCRIPTION OF THE DRAWINGS

The accompanying drawings herein are incorporated into and form a part of the specification, which illustrate embodiments consistent with the present disclosure and together with the specification serve to explain the principles of the present disclosure. Furthermore, these drawings and textual descriptions are not intended to limit the scope of the concepts of the present disclosure in any way, but rather to explain the concepts of the present disclosure to those skilled in the art by referring to specific embodiments.
FIG. 1 is a schematic diagram of a measurement electrode according to an embodiment of the present disclosure.
FIG. 2 is a schematic cross-sectional view of a structure of the measurement electrode shown in FIG. 1.
FIG. 3 is a schematic diagram of a partial structure of an electrode layer in the measurement electrode shown in FIG. 2.
FIG. 4 is a schematic diagram of a structure with a terminal installed for the measurement electrode shown in FIG. 1.

### DETAILED DESCRIPTION

The technical solutions in the embodiments of the present disclosure will be described clearly and completely below with reference to the accompanying drawings in the embodiments of the present disclosure. Obviously, the described embodiments are merely a part of the embodiments of the present disclosure, rather than all the embodiments. Based on the embodiments of the present disclosure, all other embodiments obtained by a person of ordinary skill in the art without making creative efforts shall fall within the protection scope of the present disclosure.

Embodiments of the present disclosure provide a wearable device. The wearable device may be, for example, a smart bracelet, a smart watch, a smart waist belt, smart glasses, a smart garment, or the like. The wearable device may detect the wearer's movement, sleep, and daily activities, thereby realizing the monitoring of the wearer's physical condition. The wearable device may monitor physical parameters of the wearer, such as heart rate, blood oxygen, blood pressure, or the like. The wearable device may realize the monitoring of the wearer's physical health condition through a plurality of sensors or detection circuits, such as a temperature sensor, an acceleration sensor, a gyroscope, a magnetometer, a current detection circuit, a resistance detection circuit, or the like. Among the plurality of physiological signals of the human body, an electrical signal, as the important physiological signal, may reflect a plurality of physical conditions. The electrical signal includes an electrocardiographic signal, an electromyographic signal, an electroencephalographic signal, an electrooculographic signal, or the like. The wearable device includes a measurement electrode 1. The measurement electrode 1 is configured to conform to the skin of the human body, thereby acquiring the aforementioned electrical signals.

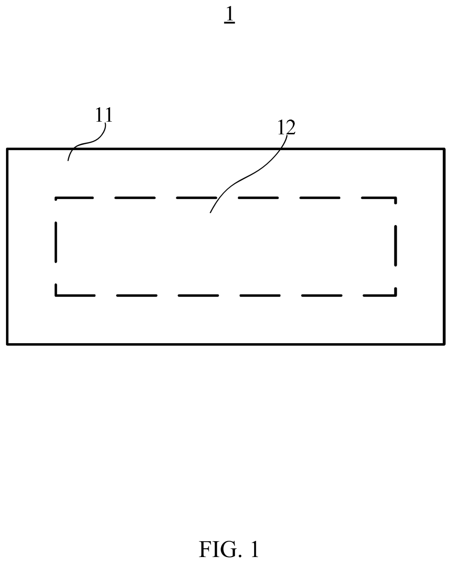

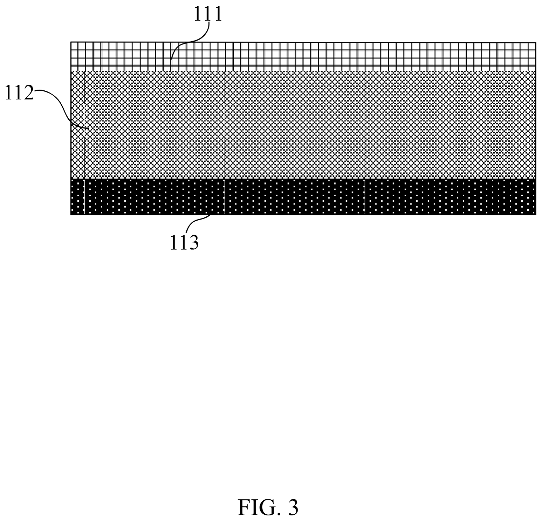

With reference to FIGs. 1-3, embodiments of the present disclosure provide the measurement electrode 1. The measurement electrode 1 includes a bottom layer 13, an electrode layer 11, and a pad 12. The bottom layer 13 and the electrode layer 11 are stacked. In a direction away from the bottom layer 13, the electrode layer 11 includes a waterproof film 113, a conductive fabric 112, and a conductive coating 111 sequentially arranged in a stacked arrangement. The pad 12 is disposed between the bottom layer 13 and the electrode layer 11, and both an area of the electrode layer 11 and an area of the bottom layer 13 are greater than an area of the pad 12. The bottom layer 13 and the electrode layer 11 sealedly cover the pad 12. In some embodiments, in a direction from the bottom layer 13 toward the pad 12, a stacking order of the electrode layer 11 is: the waterproof film 113, the conductive fabric 112, and the conductive coating 111 in sequence.

When the measurement electrode 1 is pressed against the skin of the wearer, the measurement electrode 1 is affected by sweat exuded from the skin. The conductive coating 111 can firstly play a certain waterproof role, and the waterproof film 113 can further prevent sweat from seeping into the pad 12. Such an arrangement, on the one hand, prevents sweat from affecting the electrical performance of the measurement electrode 1, and on the other hand, prevents sweat from remaining in the measurement electrode 1 and causing odor or mildew. The conductive fabric 112 has good conductivity, but the conductive fabric 112 has poor conformity with the skin. The conductive coating 111 has good conformity with the skin, but the conductive coating 111 has poor conductivity. By means of stacking the conductive fabric 112 and the conductive coating 111, the advantages of the conductive fabric 112 and the conductive coating 111 can be combined, so that the electrode layer 11 can simultaneously have good conductivity and conformity with the skin.

Furthermore, by disposing the pad 12 with a thickness greater than that of the electrode layer 11 between the electrode layer 11 and the bottom layer 13, the measurement electrode 1 can have sufficient deformation margin in the thickness direction during contact with the skin, thereby enabling the measurement electrode 1 to better conform to the skin and thus addressing the technical problem of motion artifacts generated during the measurement process.

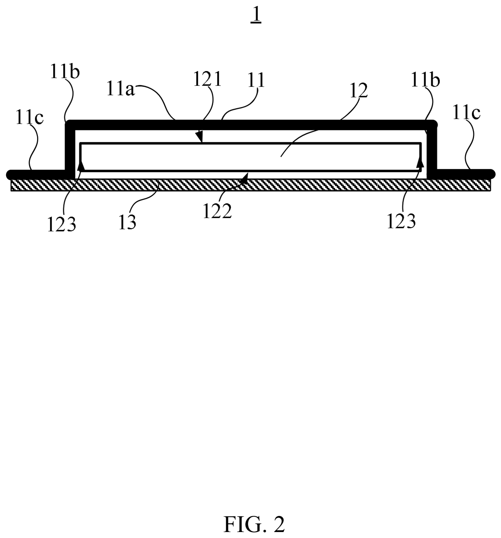

In some embodiments, with reference to FIG. 2, the pad 12 includes a first main surface 121 and a second main surface 122 disposed opposite to each other, and a side peripheral surface 123 surrounding the first main surface 121 and the second main surface 122. The electrode layer 11 includes a first covering region 11a, a second covering region 11b, and a bonding region 11c. The first covering region 11a covers the first main surface 121. The second covering region 11b covers the side peripheral surface 123. The bottom layer 13 covers the second main surface 122. The bonding region 11c is adhered to the bottom layer 13. In this way, the electrode layer 11 and the bottom layer 13 can cover the pad 12 between them, and a stepped structure is formed on the electrode layer 11, thereby facilitating the conformance of the electrode layer with the skin. The bottom layer 13 may be an independently formed encapsulation layer or a housing of another component, which is not limited herein.

In some embodiments, the conductive coating 111 is coated on a portion of the conductive fabric 112 that covers the pad 12. The pad 12 disposed between the electrode layer 11 and the bottom layer 13 causes the measurement electrode 1 to have a raised portion. The stacked structure of the raised portion is a three-layer stacked structure including the bottom layer 13, the pad 12, and the electrode layer 11. A relatively recessed portion in the measurement electrode 1 is a two-layer stacked structure of the bottom layer 13 and the electrode layer 11. For the measurement electrode 1, the portion that needs direct contact with the skin is the aforementioned raised portion with the pad 12. The conductive coating 111 is coated on the portion of the conductive fabric 112 that covers the pad 12, that is, the aforementioned raised portion of the conductive coating 111 affected by the pad 12 can meet the requirement of the measurement electrode 1 for conforming to the skin. At the same time, it can reduce the impact on the conductivity of the portion of the electrode layer 11 that is not coated with the conductive coating 111.

In some embodiments, the pad 12 is formed by at least one of room temperature vulcanized silicone rubber, space cotton, or foam. The materials have excellent properties in terms of weight, compression deformation, and elastic recovery, and can enable the measurement electrode 1 to better conform to the skin.

In some embodiments, the thickness of the pad 12 may be in a range of 0.1 mm to 10 mm, for example, 0.3 mm to 0.8 mm, 0.4 mm to 0.6 mm.

For example, the thickness of the pad 12 may be 0.35 mm, 0.45 mm, 0.55 mm, 0.65 mm, or the like. If the thickness of the pad 12 is too small, a provided deformation amount may be too small, which may result in that the conformity between the measurement electrode 1 and the skin can't be sufficiently improved. If the thickness of the pad 12 is too large, a thickness and a volume of the measurement electrode 1 may become too large, which may instead reduce the conformity between the measurement electrode 1 and the skin.

In some embodiments, a hardness of the pad 12 is between 0 degrees according to the Shore 00 standard and 90 degrees according to the Shore A standard. In the field of material technology, Shore hardness may be used to describe hardness. There are different standards for Shore hardness, for example, the Shore 00 standard, the Shore A standard, the Shore D standard, etc. A person skilled in the field may select different hardness standards based on the material to be tested, and different hardness standards may be converted to each other to some extent. The present disclosure uses different hardness standards to define a minimum hardness and a maximum hardness of the material of the pad 12. The minimum hardness of the pad 12 needs to be greater than 0 degrees according to the Shore 00 standard. The maximum hardness of the pad 12 needs to be less than 90 degrees according to the Shore A standard. If the hardness of the pad 12 is too low, the pad 12 cannot provide sufficient support for the electrode layer 11, and cannot improve the conformity between the measurement electrode 1 and the skin. If the hardness of the pad 12 is too high, a deformation that the measurement electrode 1 as a whole can generate may be reduced, which may reduce the conformity between the measurement electrode 1 and the skin.

In some embodiments, a thickness of the conductive coating 111 may be in a range of 0.01 mm to 0.5 mm, for example, 0.05 mm to 0.4 mm, 0.1 mm to 0.3 mm, or 0.15 mm to 0.25 mm. For example, the thickness of the conductive coating 111 may be 0.03 mm, 0.08 mm, 0.12 mm, 0.16 mm, 0.23 mm, 0.28 mm, 0.31 mm, 0.37 mm, 0.43 mm, 0.47 mm, or the like. If the conductive coating 111 is too thin, a durability of the conductive coating 111 may decrease, and the conductive coating 111 may be easily damaged. If the thickness of the conductive coating 111 is too large, on one hand, the volume of the measurement electrode 1 may become too large, affecting the conformity between the measurement electrode 1 and the skin; on the other hand, a resistance of the conductive coating 111 may become too large, affecting a transmission of the electrical signals.

In some embodiments, the hardness of the conductive coating 111 is in a range of 0 degrees to 50 degrees according to the Shore A standard. For example, the hardness of the conductive coating 111 may be 11 degrees, 18 degrees, 23 degrees, 28 degrees, 33 degrees, 39 degrees, 42 degrees, 47 degrees, or the like. If the hardness of the conductive coating 111 is too low, the conductive coating 111 is extremely prone to deformation, which may introduce noise into the acquisition of signals. If the hardness of the conductive coating 111 is too high, the conformity of the conductive coating 111 with the skin decreases. It should be noted that the hardness of the conductive coating 111 refers to its intrinsic hardness. Since the thickness of the conductive coating 111 on the measurement electrode 1 does not support standard hardness testing, the hardness herein refers to the hardness of a sample formed of the same material with a comparable thickness that supports standard hardness testing.

In some embodiments, the conductive coating 111 is formed by at least one of conductive resin, conductive silicone, conductive thermoplastic polyurethane, or conductive silver paste. The conductive coating 111 is configured to conform to the skin, enabling electrical signals to pass through on one hand, and achieving good conformity to the skin on the other hand.

In some embodiments, the conductive resin includes epoxy resin, conductive filler, silane coupling agent, curing agent, and accelerator. The silane coupling agent can improve a dispersibility and an adhesion of the conductive filler in the resin, enhance a compatibility between the conductive filler and the epoxy resin, improve processability, and improve mechanical, electrical, and weather resistance properties of the conductive resin. The curing agent (or cross-linking agent) may be an aliphatic amine, a thermosetting resin, an amide, a polyamide, or an acid anhydride, which is not limited herein. The curing agent can enable the epoxy resin to complete chemical reactions such as condensation, ring closure, addition, catalysis during molding, or the like. The accelerator may be an aliphatic amine accelerator, an acid anhydride accelerator, a polyetheramine catalyst, a latent catalyst, etc., which is not limited herein. The accelerator can increase a reaction rate of the conductive resin during the molding process.

In some embodiments, the conductive silicone includes silicone, conductive filler, silane coupling agent, curing agent, and accelerator. The silane coupling agent can improve a dispersibility and an adhesion of the conductive filler in the silicone, enhance a compatibility between the conductive filler and the silicone, improve processability, and improve mechanical, electrical, and weather resistance properties of the conductive silicone. The curing agent (or cross-linking agent) may be an aliphatic amine, a thermosetting resin, an amide, a polyamide, or an acid anhydride, which is not limited herein. The curing agent can enable the silicone to complete chemical reactions such as condensation, ring closure, addition, or catalysis during molding. The accelerator may be an aliphatic amine accelerator, an acid anhydride accelerator, a polyetheramine catalyst, a latent catalyst, etc., which is not limited herein. The accelerator can increase a reaction rate of the conductive silicone during the molding process.

In some embodiments, the conductive thermoplastic polyurethane includes thermoplastic polyurethane (TPU) and conductive filler.

Based on the foregoing embodiments, the conductive filler is formed by at least one of conductive carbon black, conductive graphene, or conductive carbon nanotube. The conductive filler can increase the conductivity of the conductive coating 111, which is beneficial for a transmission of the electrical signals.

In some embodiments, the conductive coating 111 may also be mixed with pigments or colorants of different colors to change a color of the conductive coating 111.

In some embodiments, the conductive silver paste includes epoxy resin, silver nanoparticles, curing agent, and accelerator. The curing agent may cause molecular chains in the epoxy resin to cross-link, promoting its curing. The accelerator can increase a reaction rate during the molding of the conductive coating 111. A person skilled in the field may select types of the curing agent and the accelerator according to actual situations, which are not repeated herein.

In some embodiments, a thickness of the conductive fabric 112 is 0.05-1 mm, e.g., 0.1 mm-0.7 mm, 0.3 mm-0.6 mm, etc. Specifically, the thickness of the conductive fabric 112 may be 0.08 mm, 0.2 mm, 0.4 mm, 0.7 mm, 0.9 mm, or the like. If the thickness of the conductive fabric 112 is too small, an overall strength of the measurement electrode 1 may decrease. If the thickness of the conductive fabric 112 is too large, the deformation that the measurement electrode 1 can generate may be reduced, affecting the conformity to the skin.

In some embodiments, the conductive fabric 112 includes at least one of conductive silver fabric, nickel-copper conductive fabric, copper conductive fabric, or stainless steel conductive fabric. The conductive fabric 112 is woven from a conductive material, providing good conductivity on one hand and having an appropriate stiffness on the other hand, thereby facilitating conformity to the skin.

In some embodiments, a thickness of the waterproof film 113 may be in a range of 10 µm to 200 µm, for example, the thickness is in a range of 50 µm-150 µm, 80 µm-120 µm. Specifically, the thickness of the waterproof film 113 may be 30 µm, 60 µm, 80 µm, 110 µm, 140 µm, 170 µm, or the like.

The waterproof film 113 is adhered to the conductive fabric 112 through a hot-pressing process, and no adhesive is provided between the waterproof film 113 and the conductive fabric 112. Since the measurement electrode 1 conformities directly to the skin, allergens within the measurement electrode 1 need to be minimized. Adhesives such as glue may easily become allergens. The present disclosure adheres the waterproof film 113 to the conductive fabric 112 through the hot-pressing process, which may reduce allergens carried in the measurement electrode 1. Similarly, bonding between the electrode layer 11 and the bottom layer 13, as well as between other layers, may also employ the hot-pressing process.

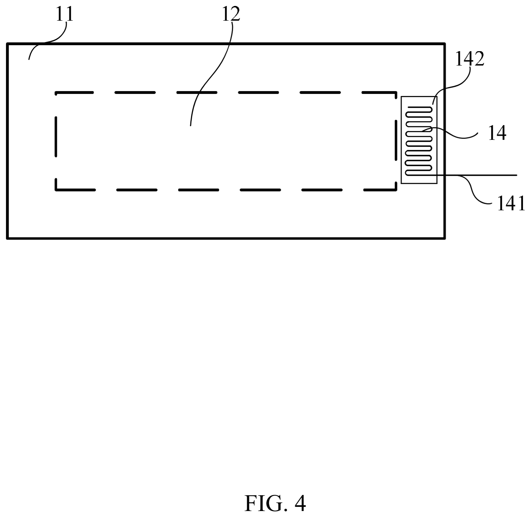

With reference to FIG. 4, in some embodiments, the measurement electrode 1 further includes a terminal 14. The terminal 14 is disposed on a portion of the electrode layer 11 that does not cover the pad 12. An electrical signal measured by the measurement electrode 1 may be output to the wearable device through the terminal 14, facilitating processing, analysis, and visualization of the electrical signal by the wearable device. The portion of the electrode layer 11 that covers the pad 12 is configured to contact the skin. Disposing the terminal 14 on the portion of the electrode layer 11 that does not cover the pad 12 may reduce an impact of the terminal 14 on the electrical signal acquisition by the measurement electrode 1.

Furthermore, the terminal 14 is disposed on a portion of the conductive fabric 112 that does not cover the pad 12. The portion of the conductive fabric 112 that does not cover the pad 12 is not provided with the conductive coating 111. In other words, the portion of the electrode layer 11 that does not cover the pad 12 has no distribution of the conductive coating 111. This enables the terminal 14 to be directly electrically connected to the conductive fabric 112 which has better conductivity, facilitating the transmission of the electrical signals.

In some embodiments, the terminal 14 includes a conductive wire 141 and a composite layer 142. The conductive wire 141 is disposed between the composite layer 142 and the conductive fabric 112. The conductive wire 141 may directly contact the conductive fabric 112, thereby outputting the electrical signal. The conductive wire 141 may be arranged in a serpentine shape, a zigzag shape, or the like, on the conductive fabric 112, thereby increasing a contact area between the conductive wire 141 and the conductive fabric 112 and reducing a resistance during the transmission of the electrical signals. The composite layer 142 may press the conductive wire 141 onto the conductive fabric 112.

In some embodiments, the conductive wire 141 includes an adhering portion and a lead-out portion. The adhering portion adheres to the electrode layer and is arranged in a serpentine arrangement. The lead-out portion is connected to the adhering portion and extends outside the electrode layer 11. The serpentine arrangement of the adhering portion may increase the contact area between the conductive wire 141 and the conductive fabric 112. The lead-out portion may facilitate a connection between the conductive wire 141 and an external device.

Furthermore, the electrode layer 11 has a length direction and a width direction. The adhering portion includes a plurality of first conductive wire segments and a plurality of second conductive wire segments. The plurality of first conductive wire segments are arranged side by side with each other along one of the length direction and the width direction and extend along the other of the length direction and the width direction. The plurality of second conductive wire segments respectively connect adjacent end portions of two adjacent first conductive wire segments.

Furthermore, the composite layer 142 includes a conductive wire layer and a thermoplastic polyurethane layer. The conductive wire layer faces the conductive wire 141. Conductive wires may be woven or otherwise formed into the conductive wire layer. The conductive wire layer may be combined with the thermoplastic polyurethane layer through hot-pressing, and then combined with the conductive wire 141 and the conductive fabric 112. In the terminal 14, the conductive wire 141 is directly electrically connected to the conductive fabric 112 on one hand, and after being pressed together with the conductive wire layer, it may be indirectly electrically connected to the conductive fabric 112 through the conductive wire layer on the other hand. This configuration further increases a contact area between the conductive wire 141 and the conductive fabric 112, reduces a resistance between the terminal 14 and the conductive fabric 112, and is beneficial for further propagation of electrical signals.

The conductive wire layer includes at least one of a conductive yarn, a conductive metal wire, or a conductive carbon fiber. If the conductive wire layer is formed by the conductive yarn, a diameter of the conductive yarn is 10 to 500 Denier. For example, the diameter of the conductive yarn may be 50 to 400 denier, 100 to 300 denier, or 150 to 250 denier. Specifically, the diameter of the conductive yarn may be 40 denier, 80 denier, 100 denier, 150 denier, 180 denier, 230 denier, 300 denier, or the like. If the conductive wire layer is formed by the conductive metal wire, a diameter of the conductive metal wire is 10 µm to 100 µm. For example, the diameter of the conductive metal wire may be 20 µm to 80 µm, 40 µm to 60 µm, or 45 µm to 55 µm. If the conductive wire layer is formed by the conductive carbon fiber, a diameter of the conductive carbon fiber is 10 µm to 100 µm. For example, the diameter of the conductive carbon fiber may be 20 µm to 80 µm, 40 µm to 60 µm, or 45 µm to 55 µm. If the conductive wires in the conductive wire layer are too thin, a contact area between the conductive wire layer and the conductive fabric 112 may be too small, failing to sufficiently optimize the connection between the conductive wire 141 and the conductive fabric 112. If the conductive wires are too thick, a thickness of the conductive wire layer may become too large, affecting the connection between the terminal 14 and the conductive fabric 112, and impacting the adhesion between the measurement electrode 1 and the skin.

The above embodiments are merely illustrative of the present disclosure and are not intended to limit the scope of the patent of the present disclosure. Any equivalent structure or equivalent process transformation made based on the contents of the specification and drawings of the present disclosure, or direct or indirect application in other related technical fields, shall be similarly included within the patent protection scope of the present disclosure.

## Claims

1. A measurement electrode, comprising:
a bottom layer and an electrode layer, wherein the bottom layer and the electrode layer are stacked; and
a pad disposed between the bottom layer and the electrode layer, wherein
both an area of the electrode layer and an area of the bottom layer are greater than an area of the pad;
the bottom layer and a waterproof film sealedly cover the pad; and
a thickness of the pad is greater than a thickness of the electrode layer.

2. The measurement electrode according to claim 1, wherein
the pad comprises a first main surface and a second main surface disposed opposite to each other, and a side peripheral surface connecting the first main surface and the second main surface; and
the electrode layer comprises a first covering region, a second covering region, and a bonding region, wherein the first covering region covers the first main surface, the second covering region covers the side peripheral surface, the bottom layer covers the second main surface, and the bonding region is bound with the bottom layer.

3. The measurement electrode according to claim 1, wherein
in a direction away from the bottom layer, the electrode layer comprises the waterproof film, a conductive fabric, and a conductive coating sequentially arranged in a stacked arrangement.

4. The measurement electrode according to claim 3, wherein
a hardness of the conductive coating is between 0 degrees and 50 degrees according to the Shore A standard;
a thickness of the conductive coating is 0.01 mm to 0.5 mm; and
a thickness of the conductive fabric is 0.05 mm to 1 mm.

5. The measurement electrode according to claim 3, wherein the conductive coating is formed by at least one of conductive resin, conductive silicone, conductive thermoplastic polyurethane, or conductive silver paste.

6. The measurement electrode according to claim 5, wherein
the conductive resin comprises epoxy resin, conductive filler, silane coupling agent, curing agent, and accelerator;
the conductive silicone comprises silicone, conductive filler, silane coupling agent, curing agent, and accelerator;
the conductive thermoplastic polyurethane comprises thermoplastic polyurethane and conductive filler; and
the conductive silver paste comprises epoxy resin, silver nanoparticles, curing agent, and accelerator.

7. The measurement electrode according to claim 6, wherein the conductive filler is formed by at least one of conductive carbon black, conductive graphene, or conductive carbon nanotube.

8. The measurement electrode according to claim 1, wherein the conductive coating is coated on a portion of the conductive fabric that covers the pad.

9. The measurement electrode according to claim 1, wherein the conductive fabric includes at least one of conductive silver fabric, nickel-copper conductive fabric, copper conductive fabric, or stainless steel conductive fabric.

10. The measurement electrode according to claim 1, wherein a thickness of the waterproof film is 10 µm to 200 µm.

11. The measurement electrode according to claim 1, wherein the waterproof film is adhered to the conductive fabric through a hot-pressing process, and no adhesive is provided between the waterproof film and the conductive fabric.

12. The measurement electrode according to claim 1, wherein the measurement electrode further comprises a terminal, wherein the terminal is disposed on a portion of the electrode layer that does not cover the pad.

13. The measurement electrode according to claim 12, wherein
the terminal is disposed on a portion of the conductive fabric that does not cover the pad;
the portion of the conductive fabric that does not cover the pad is not provided with the conductive coating; and
the terminal comprises a conductive wire and a composite layer, wherein the conductive wire is disposed between the composite layer and the conductive fabric.

14. The measurement electrode according to claim 13, wherein
the conductive wire comprises an adhering portion and a lead-out portion, wherein the adhering portion adheres to the electrode layer and is arranged in a serpentine arrangement, and the lead-out portion is connected to the adhering portion and extends outside the electrode layer.

15. The measurement electrode according to claim 13, wherein the composite layer comprises a conductive wire layer and a thermoplastic polyurethane layer, the conductive wire layer facing the conductive wire.

16. The measurement electrode according to claim 1, wherein
the conductive wire layer includes at least one of a conductive yarn, a conductive metal wire, or a conductive carbon fiber, wherein a diameter of the conductive yarn is 10 to 500 Denier, a diameter of the conductive metal wire is 10 µm to 100 µm, or a diameter of the conductive carbon fiber is 10 µm to 100 µm.

17. The measurement electrode according to claim 1, wherein the pad is formed by at least one of room temperature vulcanized silicone rubber, space cotton, or foam.

18. The measurement electrode according to claim 1, wherein
the thickness of the pad is 0.1 mm to 10 mm; and
a hardness of the pad is between 0 degrees according to the Shore 00 standard and 90 degrees according to the Shore A standard.

19. A wearable device, comprising the measurement electrode according to any one of claims 1-18.
